# EUROPÄISCHE PATENTSCHRIFT

(11) **EP 1 152 684 B1**
(45) Veröffentlichungstag und Bekanntmachung des Hinweises auf die Patenterteilung: **17.12.2003**
(21) Anmeldenummer: 99906240.9
(22) Anmeldetag: 18.02.1999
(51) Int. Cl.: A61B 1/12

(54) **ENDOSKOP**
ENDOSCOPE
ENDOSCOPE

(43) Veröffentlichungstag der Anmeldung: 14.11.2001
(73) Patentinhaber: Karl Storz GmbH & Co. KG, 78532 Tuttlingen (DE)
(72) Erfinder: RUDISCHHAUSER, Jürgen, D-78532 Tuttlingen (DE); HÖFIG, Siegfried, D-78532 Tuttlingen (DE)
(74) Vertreter: Heuckeroth, Volker, Dr.
(86) Internationale Anmeldenummer: EP9901018
(87) Internationale Veröffentlichungsnummer: WO00048505

(56) Entgegenhaltungen:
- WO-A-94/11052
- US-A- 4 867 138
- US-A- 5 048 508

## Beschreibung

Die Erfindung betrifft ein Endoskop, mit einem Außenschaft, in dem ein ein optisches System aufnehmender Optikkanal angeordnet ist, wobei in dem Außenschaft weiterhin ein Arbeitskanal zur Aufnahme eines Arbeitselementes und zumindest ein Kanal zur Aufnahme eines lichtzuführenden Beleuchtungssystems angeordnet sind, wobei der Optikkanal und der zumindest eine Kanal für das lichtzuführende Beleuchtungssystem als Kammern eines im Querschnitt gekammerten Profilschafts ausgebildet sind, wobei der Profilschaft im Außenschaft aufgenommen ist, und wobei eine Außenkontur des Profilschafts etwa nierenförmig ausgebildet ist, und der Arbeitskanal innerhalb des Außenschafts und außerhalb des Profilschafts angeordnet ist und sich von einer konkaven Außenseite des Profilschafts bis zu einer gegenüberliegenden konkaven Innenseite des Außenschafts erstreckt, wobei ferner in dem Außenschaft zumindest ein sich längs erstreckender Zuführkanal für ein flüssiges Medium vorhanden ist.

Ein derartiges Endoskop ist aus der US-A-4 867 138 bekannt.

Ein Endoskop der eingangs genannten Art wird in der minimal-invasiven Chirurgie zur Durchführung von Operationen im menschlichen oder tierischen Körper unter gleichzeitiger Sichtkontrolle verwendet.

Der Außenschaft eines solchen Endoskops ist daher dazu ausgelegt, neben der Beobachtungsoptik auch ein Arbeitselement, beispielsweise eine Präparierzange, aufzunehmen, das vom proximalen Ende her durch den Außenschaft einführbar ist und während der Operation sicher gehalten wird. Die Maulteile eines derartigen Arbeitselements ragen dann aus dem distalen offenen Ende des Außenschafts hervor und können zur Durchführung der Operation mittels einer Handhabe am proximalen Ende betätigt werden.

Um mit dem Endoskop eine Operation durch eine möglichst kleine künstlich geschaffene Inzision in der Körperdecke hindurch durchführen zu können, besteht eine Anforderung an ein solches Endoskop darin, daß der Außenschaft einen möglichst kleinen Durchmesser aufweist. Da während einer Operation mit demselben Endoskop mehrere verschiedene Arbeitselemente verwendet werden, die sich im Durchmesser unterscheiden können, wird der im Inneren des Außenschafts für den Optikkanal verbleibende Querschnitt durch den maximalen Durchmesser des größten verwendeten Arbeitselementes begrenzt. Um eine optimale Sichtkontrolle der Operation mit großem Blickfeld und hoher Bildqualität zu ermöglichen, ist es jedoch wünschenswert, ein optisches System mit möglichst großem Querschnitt zu verwenden. An dieser Stelle sei bemerkt, daß unter einem optischen System im Sinne der vorliegenden Erfindung jede Art von Bildübertragungssystemen verstanden wird, bspw. ein Relaislinsensystem, Glasfaser-Bildleiter oder ein Videokabel für einen distal angeordneten Videochip.

Des weiteren ist in einem derartigen Endoskop zumindest ein Zuführkanal für ein Medium, beispielsweise eine Spülflüssigkeit, sowie ein von der Zuführung getrennter Abführkanal zur Abführung eines Mediums, beispielsweise zur Abführung von Blut, Geweberesten zusammen mit der eingeleiteten Spülflüssigkeit vorhanden. Eine solche Anordnung ermöglicht während der Operation ein kontinuierliches Spülen und Absaugen, damit die Sichtkontrolle durch die Optik während der Operation nicht durch Blut oder lose Gewebestücke beeinträchtigt wird.

Bei einem wird von der Firma Karl Storz GmbH & Co., Tuttlingen, als Kombinationsinstrument bspw. unter der Instrumentennummer 27092 AM - 27093 B - 27093 BI vertriebenen Endoskop ist innerhalb des Außenschaftes zunächst ein Innenschaft angeordnet, wobei innerhalb des Innenschaftes der Optikkanal sowie der Arbeitskanal zur Aufnahme des Arbeitselementes ausgebildet ist. Der Innenschaft weist im Querschnitt etwa eine dreieckige Form auf, so daß zwischen der Innenseite des Außenschaftes und der Außenseite des Innenschaftes ein Abführkanal für die Abführung eines Mediums und innerhalb des Innenschaftes zwischen dem Optikkanal und dem Arbeitskanal für das Arbeitselement ein Zuführkanal für ein Medium gebildet wird. Der Optikkanal, in dem das optische System angeordnet ist, wird durch einen weiteren Optikschaft gebildet. Mit dem das optische System aufnehmenden Optikschaft sind ferner noch zwei jeweils ein lichtzuführendes System in Form von Lichtleitfasern aufnehmende Rohrschäfte von ebenfalls rundem Querschnitt verbunden.

Bei diesem bekannten Endoskop ist es als nachteilig anzusehen, daß der im Inneren des Außenschaftes für den Optikkanal vorhandene Querschnitt durch den zusätzlichen Innenschaft, der bei dieser Ausgestaltung erforderlich ist, um einen Zuführkanal und einen davon getrennten Abführkanal zu bilden, vermindert ist. Eine weitere Einschränkung des nutzbaren Raumes innerhalb des Außenschaftes wird dadurch hervorgerufen, daß das Arbeitselement ebenfalls in dem Innenschaft aufgenommen ist. Diese Anordnung führt dazu, daß für den Optikkanal und damit für das optische System nur ein verminderter Querschnitt zur Verfügung steht, woraus der zuvor erwähnte Nachteil eines geringeren Blickfeldes und einer geringeren Bildqualität resultiert.

Ein weiteres derartiges Endoskop ist aus der DE-Firmenschrift der Firma Richard Wolf GmbH, Knittlingen, "Ambulanz-Dauerspül-Operations-Hysteroskop nach Grochmal", bekannt. Bei diesem Endoskop sind innerhalb des Außenschaftes vier im Querschnitt runde Rohrschäfte angeordnet. Ein erster Rohrschaft bildet den Kanal zur Aufnahme des Arbeitselements, ein zweiter Rohrschaft bildet den Zuführkanal, ein dritter Rohrschaft den Abführkanal sowie ein vierter Rohrschaft den Optikkanal. Der zwischen den einzelnen Rohrschäften und der Innenseite des Außenschafts verbleibende Raum ist mit Lichtleitfasern aufgefüllt, die mit dem Außenschaft verkittet sind.

Auch bei diesem Endoskop wird der innerhalb des Außenschaftes zur Verfügung stehende Raum nicht optimal genutzt, da zur Aufnahme des Arbeitselementes ein zusätzlicher innerer Rohrschaft vorgesehen ist. Dieser Rohrschaft ist jedoch erforderlich, um den Arbeitskanal von den Lichtleitfasern zu trennen, damit diese beim Einführen des Arbeitselementes nicht beschädigt werden.

Ein weiterer Nachteil dieses Endoskops besteht darin, daß der Außenschaft von den innen angeordneten Rohrschäften wegen der dazwischen liegenden Lichtleitfasern nicht abnehmbar ist.

Aus dem Dokument US-A-4 867 138 ist ein Endoskop bekannt, das einen Schaft aufweist, der durch einen Außenschaft und einen als Profilschaft ausgebildeten Innenschaft gebildet wird. In dem Innenschaft sind insgesamt drei Kammern ausgebildet, von denen eine mittlere Kammer elektrische Kabel des optischen Systems, das eine Videokamera am distalen Ende des Endoskops umfaßt, aufnimmt, während in den beiden seitlich dieser den Optikkanal bildenden Kammern jeweils eine Kammer zur Aufnahme des Beleuchtungssystems in Form von Lichtleitfasern angeordnet ist. Der Innenschaft ist insgesamt nierenförmig ausgebildet, wobei der lichte Raum zwischen der konkaven Seite des Profilschafts und der gegenüberliegenden konkaven Innenseite des Außenschafts einen Arbeitskanal zur Aufnahme eines Arbeitselements bildet. Dieser Arbeitskanal dient bei dem'bekannten Endoskop gleichzeitig als Zuführkanal für ein flüssiges Medium, d.h. beispielsweise als Spülkanal für eine Spülflüssigkeit. Dieses bekannte Endoskop kann nicht zum gleichzeitigen Spülen und Absaugen verwendet werden. Bei einem weiteren aus diesem Dokument beschriebenen Ausführungsbeispiel eines Endoskops wird vorgeschlagen, einen Zuführkanal und einen davon getrennten Abführkanal dadurch zu realisieren, daß der Profilschaft auf seiner dem Arbeitskanal gegenüberliegenden Seite nicht formschlüssig an die Innenkontur des Außenschaftes angepaßt wird, so daß zwischen dieser Seite des Profil schaftes und dieser Innenseite des Außenschaftes ein Flüssigkeitskanal gebildet wird. Eine sichere Trennung des Zuführkanals von dem Abführkanal ist dadurch nicht gewährleistet.

Schließlich ist aus dem Dokument WO-A-94/11052 ein Endoskop bekannt, bei dem der innere Schaft den gesamten Innenraum des Außenschafts ausfüllt, wobei im Innenschaft eine Mehrzahl von Bohrungen zur Realisierung des Optikkanals, des Arbeitskanals und eines Spül-/Saugkanals ausgebildet sind. Der Arbeitskanal ist dabei ebenfalls als Bohrung in dem Innenschaft realisiert und befindet sich somit innerhalb des Innenschaftes.

Der Erfindung liegt die Aufgabe zugrunde, ein Endoskop der eingangs genannten Art dahingehend weiterzubilden, daß die Anordnung aus Optikkanal, Arbeitskanal zur Aufnahme des Arbeitselementes und Zuführkanal eine optimale Raumausnutzung des Innenraums des Außenschaftes gewährleistet, so daß der für den Optikkanal zur Verfügung stehende Querschnitt möglichst groß ist, und daß außerdem ein gleichzeitiges Saugen und Spülen möglich ist.

Erfindungsgemäß wird diese Aufgabe hinsichtlich des eingangs genannten Endoskops dadurch gelöst, daß in dem Außenschaft zumindest ein sich längs erstreckender Abführkanal zur von der Zuführung getrennten Abführung eines flüssigen Mediums vorhanden ist, und daß der Profilschaft zwei weitere Kammern aufweist, die den sich längs erstreckenden Zuführkanal und/oder den sich längs erstreckenden Abführkanal bilden.

Zur Ausbildung des Optikkanals und des zumindest einen Zuführkanals und/oder des zumindest einen Abführkanals ist bei dem erfindungsgemäßen Endoskop ein im Querschnitt gekammerter Profilschaft in dem Außenschaft aufgenommen, der zur Ausbildung der einzelnen Kanäle eine entsprechende Anzahl von Kammern oder Segmente, die im Kontext der vorliegenden Erfindung als gleichbedeutend mit Kammern verstanden werden, aufweist. Das optische System ist dabei in einer der Kammern aufgenommen, während der zumindest eine Zuführkanal und/oder der zumindest eine Abführkanal durch zumindest eine weitere Kammer des Profilschaftes gebildet wird. Durch die Anordnung des Optikkanals und zumindest des Zuführkanals oder des Abführkanals als Kammern des Profilschaftes wird ein weiterer Innenschaft wie im Stand der Technik eingespart, so daß der durch das größte Arbeitselement vorgegebene, für den Optikkanal zur Verfügung stehende Querschnitt die größtmögliche Abmessung aufweisen kann. Ein Profilschaft hat insgesamt den Vorteil einer optimalen Raumausnutzung mit jeweils maximal möglichem Querschnitt der die einzelnen Kanäle bildenden Kammern, während eine Anordnung einzelner zylindrischer Rohrschäfte selbst bei dichtester Packung nicht nutzbare Zwischenräume bildet. Ein weiterer Vorteil eines Profilschafts besteht in einer höheren Biegesteifigkeit und damit in einem besseren Schutz der Endoskopieoptik.

Zur Bildung des Arbeitskanals zur Aufnahme des Arbeitselements wird ein weiterer Rohrschaft, wie er im Stand der Technik vorgesehen ist, eingespart, so daß der verfügbare Durchmesser innerhalb des Außenschaftes um die doppelte Wanddicke eines solchen zusätzlichen Rohrschafts vergrößert ist. Zwischen der konkaven Außenseite des Profilschafts und der gegenüberliegenden Innenseite des Außenschafts wird das Arbeitselement außerdem beim Einschieben in den Außenschaft sicher geführt und während der Operation sicher gehalten.

Nicht nur das optische System, sondern auch das Licht zuführende Beleuchtungssystem, beispielsweise in Form von Lichtleitfasern, kann ebenfalls in dem Profilschaft mit größtmöglichem Querschnitt ausgebildet werden, wodurch eine helle Ausleuchtung des Operationsgebiets erreicht wird. während bei dem aus der DE-Firmenschrift der Richard Wolf GmbH, Knittlingen, bekannten Endoskop die Lichtleitfasern den zwischen dem Außenschaft und den Innenschäften verbleibenden Raum ausfüllen, wodurch die Gesamtanordnung aus Außenschaft, Lichtleitfasern und Innenschäften unzerlegbar sind, wird durch die vorliegende Erfindung außerdem vorteilhafterweise erreicht, daß die Anordnung aus Außenschaft und Profilschaft mit den darin integrierten Lichtleitfasern zerlegbar ausgestaltet werden kann.

In einer bevorzugten Ausgestaltung weist der Außenschaft einen kreisförmigen Querschnitt auf und erstreckt sich eine konvexe Außenseite des Profilschafts, die an dem Außenschaft anliegt, bis über die Hälfte des Innenumfangs des Außenschafts.

Diese Maßnahme hat den Vorteil, daß der Profilschaft in dem Außenschaft ohne zusätzliche Befestigungsmaßnahmen in dem Außenschaft lagefest gehalten wird, auch wenn das Arbeitselement noch nicht in den Außenschaft eingeführt ist. Dadurch, daß keine weitere Befestigung des Profilschafts in dem Außenschaft erforderlich ist, eröffnet dies ferner die vorteilhafte Möglichkeit, den Außenschaft von dem Profilschaft abnehmbar auszugestalten, wodurch dann eine leichtere und gründlichere Reinigung des Endoskops ermöglicht wird.

In einer weiteren bevorzugten Ausgestaltung sind einzelne der Kammern im Querschnitt nicht kreisförmig.

Diese Maßnahme hat den Vorteil, daß der Profilschaft mit einer besonders hohen Biegesteifigkeit ausgebildet werden kann, wodurch besonders das optische System in dem Optikkanal gegen Bruch geschützt ist. Da der Optikkanal wegen des darin aufgenommenen optischen Systems üblicherweise im Querschnitt rund ist, kann beispielsweise die den Zuführkanal oder die Abführkanal bildende Kammer unrund ausgebildet sein. Bei dem aus der DE-Firmenschrift der Firma Richard Wolf GmbH, Knittlingen, bekannten Endoskop, bei dem die einzelnen Kanäle durch zylindrische dünne Schäfte gebildet werden, ist die Biegefestigkeit im Unterschied zu der erfindungsgemäßen Ausgestaltung reduziert.

In einer weiteren bevorzugten Ausgestaltung ist der Profilschaft aus dem Außenschaft herausnehmbar.

Hierbei ist von Vorteil, daß einerseits die Reinigbarkeit des Endoskops verbessert ist, andererseits mehrere Außenschäfte bereitgehalten werden können, die gegeneinander bei Verwendung desselben Profilschaftes ausgetauscht werden können. Durch Verwendung unterschiedlicher Außenschäfte können dadurch falls erforderlich unterschiedlich große Arbeitskanäle zur Aufnahme des Arbeitselements realisiert werden.

In einer weiteren bevorzugten Ausgestaltung weisen der Optikkanal und der Arbeitskanal einen kreisförmigen Querschnitt auf und sind ein Durchmesser der den Optikkanal bildenden Kammer und ein Durchmesser des Arbeitskanals für das Arbeitselement auf dem gleichen Durchmesser des Außenschafts angeordnet.

Bei dieser Ausgestaltung und Anordnung des Profilschafts kann die den Optikkanal bildende Kammer vorteilhafterweise mit einem Außendurchmesser ausgebildet werden, der etwa gleich der Differenz aus dem Innendurchmesser des Außenschafts und dem Außendurchmesser des Arbeitselements ist, wodurch eine optimale Ausnutzung des für den Optikkanal maximal zur Verfügung stehenden Querschnitts erreicht wird.

In einer weiteren bevorzugten Ausgestaltung weist der Profilschaft eine solche äußere Abmessung auf, daß zwischen dem Arbeitskanal, der Innenseite des Außenschafts und dem Profilschaft zumindest ein weiterer Abführkanal und/oder Zuführkanal für ein Medium ausgebildet ist.

Bei dieser Ausgestaltung kann der Profilschaft beispielsweise eine Kammer aufweisen, die den Zuführkanal für ein Medium bildet, während der zwischen dem Arbeitselement, der Innenseite des Außenschafts und dem Profilschaft verbleibende freie Raum dann als Abführkanal für ein Medium dient. Auf diese Weise wird vorteilhaft der gesamte innere Querschnitt des Außenschafts funktionell genutzt.

In einer weiteren bevorzugten Ausgestaltung weist der Profilschaft im Querschnitt fünf Kammern auf.

Bei dieser Ausgestaltung des Profilschafts kann beispielsweise eine Kammer zur Bildung des Optikkanals mit größtmöglichem Querschnitt vorgesehen sein, während die übrigen vier Kammern beispielsweise den Zuführkanal, den Abführkanal sowie einen oder zwei Kanäle für das Licht zuführende Beleuchtungssystem bilden können.

In einer weiteren bevorzugten Ausgestaltung ist der Profilschaft einstückig ausgebildet.

Hierbei ist von Vorteil, daß der Profilschaft in einem einzigen Arbeitsgang mit der gewünschten Formgebung hergestellt werden kann, wodurch der Herstellungs- bzw. der Kostenaufwand des Profilschafts vorteilhaft reduziert wird.

Alternativ dazu kann es jedoch auch bevorzugt sein, wenn der Profilschaft aus einer der Anzahl an Kammern entsprechenden Anzahl von Einzelprofilen zusammengefügt ist.

Auch diese Bildung des Profilschafts ist vorteilhaft, da sich der Profilschaft auf diese weise in der Art eines Baukastensystems zu der gewünschten Anordnung der einzelnen Kammern zusammensetzen läßt.

Weitere Vorteile ergeben sich aus der nachfolgenden Beschreibung und der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden hiernach in Bezug auf diese näher beschrieben. Es zeigen:
- Fig. 1: eine Seitenansicht eines Endoskops in einer Gesamtdarstellung;
- Fig. 2: einen Schnitt durch das Endoskop entlang der Linie II-II in Fig. 1 in vergrößertem Maßstab;
- Fig. 3: einen der Fig. 2 entsprechenden Querschnitt gemäß einem weiteren Ausfürungsbeispiel; und
- Fig. 4: einen der Fig. 2 entsprechenden Querschnitt gemäß einem noch weiteren Ausführungsbeispiel.

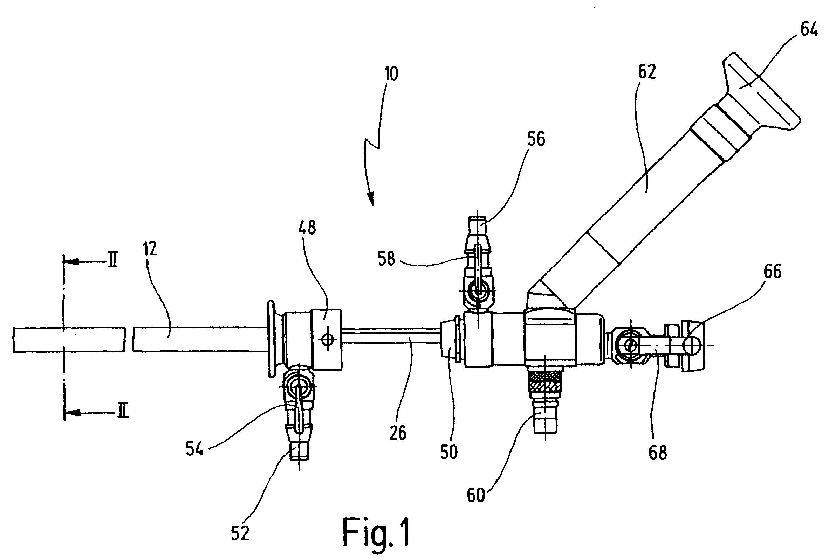

In Fig. 1 ist ein mit dem allgemeinen Bezugszeichen 10 versehenes Endoskop dargestellt. Das Endoskop 10 dient im Rahmen der minimal-invasiven Chirurgie zur Durchführung von operativen Eingriffen im menschlichen oder tierischen Körper unter Sichtkontrolle.

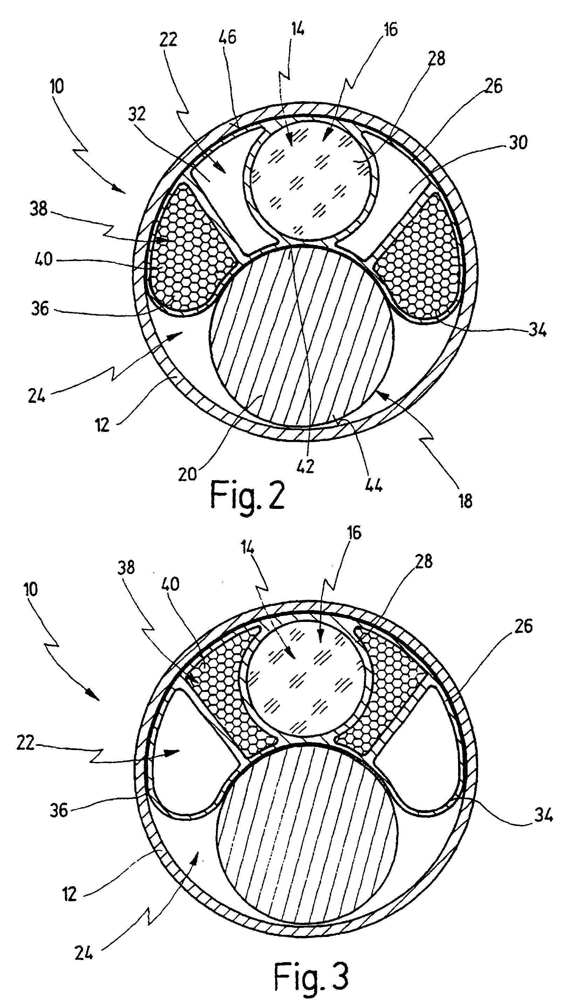

Das Endoskop 10 weist einen lang erstreckten Außenschaft 12 auf. Gemäß Fig. 2, die einen Querschnitt entlang der Linie II-II durch das Endoskop 10 in Fig. 1 in einem vergrößerten Maßstab zeigt, ist in dem Außenschaft 12 ein Optikkanal 14 angeordnet, der ein schematisch dargestelltes optisches System 16, das beispielsweise ein Relaislinsensystem umfaßt, aufnimmt.

In dem Außenschaft 12 ist weiterhin ein Arbeitskanal 18 zur Aufnahme eines schematisch dargestellten Arbeitselements 20 vorhanden. Das Arbeitselement 20 ist beispielsweise eine Präparierzange, mit der der operative Eingriff vorgenommen wird.

Des weiteren ist in dem Außenschaft 12 ein sich längs erstreckender Zuführkanal 22 für ein Medium vorhanden, beispielsweise für eine Spülflüssigkeit, die durch den Zuführkanal 22 in das Operationsgebiet eingeleitet wird. Weiterhin ist in dem Außenschaft 12 ein Abführkanal 24 für ein Medium vorhanden, über den beispielsweise die in das Operationsgebiet eingeleitete Spülflüssigkeit zusammen mit Blut oder Gewebestücken aus dem Operationsgebiet getrennt von der Zuführung der Spülflüssigkeit abgesaugt werden kann, so daß kontinuierlich gespült und abgesaugt werden kann.

Der Optikkanal 14 sowie der Zuführkanal 22 für ein Medium sind als Kammern eines im Querschnitt gekammerten Profilschaftes 26 ausgebildet, der in dem Außenschaft 18 aufgenommen ist.

Eine erste mittlere Kammer 28, die den Optikkanal 14 bildet, ist im Querschnitt rund ausgebildet. Zwei sich an die mittlere Kammer 28 anschließende Kammern 30 und 32 bilden gemeinsam den Zuführkanal 22 für die Zuführung eines Mediums. Die Kammern 30 und 32 sind im Querschnitt unrund ausgebildet.

Der Profilschaft 26 weist zwei weitere äußere Kammern 34 und 36 auf, in denen jeweils ein Licht zuführendes Beleuchtungssystem 38 aufgenommen ist, das durch einzelne Lichtleitfasern 40 gebildet wird. Die Lichtleitfasern 40 füllen die Kammer 34 bzw. die Kammer 36 vollständig aus.

Wie aus Fig. 2 hervorgeht, ist eine Außenkontur des Profilschafts 26 etwa nierenförmig ausgebildet, wobei eine konkave Außenseite 42 des Profilschafts 26 mit einer gegenüberliegenden Innenseite 44 des Außenschafts 12 den Arbeitskanal 18 zur Aufnahme des Arbeitselements 20 bildet. Zwischen der konkaven Außenseite 42 und der dieser gegenüberliegenden Innenseite 44 ist das Arbeitselement 20 geführt und sicher gehalten. Zur Bildung des Arbeitskanals 18 zur Aufnahme des Arbeitselements 20 ist somit kein weiterer Schaft erforderlich.

Die den Optikkanal 14 bildende Kammer 28 ist in dem Profilschaft 26 so angeordnet, daß ein Durchmesser der Kammer 28 und ein Durchmesser des Kanals 18 für das Arbeitselement 20 auf dem gleichen Durchmesser des Außenschafts 12 angeordnet sind. Dadurch wird für den Optikkanal 14 der größtmögliche Querschnitt bei vorgegebenem Querschnitt des Arbeitselements 20 erreicht, so daß das optische System 16 mit maximalem Durchmesser ausgebildet werden kann, wodurch wiederum die Bildqualität der Sichtbeobachtung des Operationsvorganges verbessert wird.

Der Abführkanal 24 wird durch den verbleibenden Raum zwischen dem Profilschaft 26, genauer gesagt zwischen den Kammern 34 und 36, und dem Arbeitselement 20 gebildet.

Der Profilschaft 26 weist aufgrund seiner Formgebung eine hohe Biegesteifigkeit auf.

Eine konvexe Außenseite 46 des Profilschafts 26 liegt an dem Außenschaft 12 an und erstreckt sich bis über die Hälfte des Innenumfangs des Außenschafts 12 hinaus. Dadurch wird erreicht, daß ohne zusätzliche Befestigung der Profilschaft 26 in dem Außenschaft 12 lagefest fixiert ist, auch wenn das Arbeitselement 20 nicht in den Arbeitskanal 18 eingeführt ist.

Wieder mit Bezug auf Fig. 1 ist der Profilschaft 26 aus dem Außenschaft 12 herausnehmbar, bzw. der Außenschaft 12 von dem Profilschaft 26 abziehbar. In Fig. 1 ist der Außenschaft 12 in einer geringfügig gegenüber dem Profilschaft 26 verschobenen Lage gezeigt. Im Betrieb des Endoskops 10 ist jedoch der Außenschaft 12 über eine Bajonettkupplung, bestehend aus einer mit dem Außenschaft 12 verbundenen Fassung 48 und einer mit dem Profilschaft 26 verbundenen Fassung 50, fest verbunden.

An dem Außenschaft 12 ist ferner ein Anschluß 52 zum Anschließen einer nicht dargestellten Saugleitung angeordnet, die mit einer Saugvorrichtung in Verbindung steht. Der Anschluß 52 kommuniziert mit dem Abführkanal 24. Ein Absperrhahn 54 dient zum Öffnen und Absperren des Abführkanals 24.

Am proximalen Ende des Profilschafts 26 ist ferner ein Anschluß 56 zum Anschließen einer nicht dargestellten Leitung für die Zuführung eines Mediums, beispielsweise eine Spülflüssigkeitsleitung, angeordnet. Der Anschluß 56 kommuniziert mit dem Zuführkanal 22, d.h. genauer mit den Kammern 30 und 32 des Profilschafts 26. Auch der Anschluß 56 weist einen Absperrhahn 58 auf.

Ein Lichtleiteranschluß 60 dient zum Anschließen eines Licht zuführenden Lichtleitkabels, um Licht von einer externen Lichtquelle in die Lichtleitfasern 40 in den Kammern 34 und 36 des Profilschafts 26 einzukoppeln.

Ein Okulartubus 62 mit einer Okularmuschel 64 dient zur Beoachtung des durch das optische System 16 abgebildete Operationsgebiet.

Schließlich ist am proximalen Ende des Endoskops 10 in Verlängerung des Arbeitskanals 18 eine Kupplung 66 zum Einführen des in Fig. 1 nicht dargestellten Arbeitselements 20 angeordnet, wobei das Arbeitselement 20 nach dem Einführen mittels einer Verriegelung 68 fixiert werden kann.

Der Profilschaft 26 ist insgesamt einstückig ausgebildet. Der Profilschaft 26 kann jedoch auch aus Einzelprofilen zusammengefügt sein, wobei dann jedes Einzelprofil eine der Kammern 28 bis 36 bildet.

In Fig. 3 ist ein gegenüber Fig. 2 geringfügig abgewandeltes Ausführungsbeispiel dargestellt, bei dem das Licht zuführende Beleuchtungssystem 38 in den der Kammer 28, die wiederum den Optikkanal 14 bildet, benachbarten Kammern 30 und 32 aufgenommen ist, während die Kammern 34 und 36 den Zuführkanal 22 für ein Medium bilden. Das Querschnittsprofil des Profilschafts 26 ist in dem in Fig. 3 gezeigten Ausführungsbeispiel mit dem in Fig. 2 gezeigten Ausführungsbeispiel identisch.

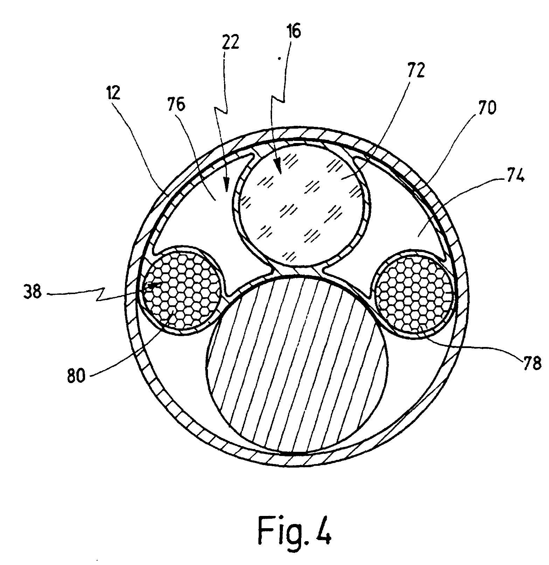

In Fig. 4 ist ein weiteres Ausführungsbeispiel eines Endoskops dargestellt, das sich von den vorhergehenden Ausführungsbeispielen durch einen Profilschaft 70 unterscheidet, der zwar wiederum fünf Kammern 72 bis 80 aufweist, die jedoch ein gegenüber den vorherigen Ausführungsbeispielen unterschiedliches Querschnittsprofil aufweisen. So weisen die Kammern 78 und 80, die das Beleuchtungssystem 38 aufnehmen, einen runden Querschnitt auf, ebenso wie die Kammer 72, die das optische System 16 aufnimmt.

Die Kammern 74 und 76 bilden gemeinsam den Zuführkanal 22.

Während die zuvor beschriebenen Ausführungsbeispiele bevorzugt sind, versteht es sich jedoch, daß beispielsweise in Fig. 2 der Zuführkanal 22 nur durch die Kammer 32 des Profilschafts 26 gebildet werden kann, während die Kammer 30 des Profilschafts 26 auch den Abführkanal 24 oder einen weiteren zusätzlichen Abführkanal bilden kann. Des weiteren ist es möglich, daß die Kammer 32 den Zuführkanal für eine Flüssigkeit und die Kammer 30 den Zuführkanal für ein Gas bilden kann.

Weiterhin versteht es sich, daß die Querschnittsprofile der einzelnen Kammern des Profilschafts 26 bzw. des Profilschafts 70 im Ermessen des Fachmanns den jeweiligen Anforderungen entsprechend angepaßt werden können.

## Patentansprüche

1. Endoskop, mit einem Außenschaft (12), in dem ein ein optisches System (16) aufnehmender Optikkanal (14) angeordnet ist, wobei in dem Außenschaft (12) weiterhin ein Arbeitskanal (18) zur Aufnahme eines Arbeitselementes (20) und zumindest ein Kanal zur Aufnahme eines lichtzuführenden Beleuchtungssystems (38) angeordnet sind, wobei der Optikkanal (14) und der zumindest eine Kanal für das lichtzuführende Beleuchtungssystem (38) als Kammern (28, 34, 36; 72, 78, 80) eines im Querschnitt gekammerten Profilschafts (26; 70) ausgebildet sind, wobei der Profilschaft (26; 70) im Außenschaft (12) aufgenommen ist, und wobei eine Außenkontur des Profilschafts (26; 70) etwa nierenförmig ausgebildet ist, und der Arbeitskanal (18) innerhalb des Außenschafts (12) und außerhalb des Profilschafts (26; 70) angeordnet ist und sich von einer konkaven Außenseite (42) des Profilschafts (26; 70) bis zu einer gegenüberliegenden konkaven Innenseite (44) des Außenschafts (12) erstreckt, wobei ferner in dem Außenschaft (12) zumindest ein sich längs erstreckender Zuführkanal (22) für ein flüssiges Medium vorhanden ist, **dadurch gekennzeichnet, daß** in dem Außenschaft (12) zumindest ein sich längs erstreckender Abführkanal (24) zur von der Zuführung getrennten Abführung eines flüssigen Mediums vorhanden ist, und daß der Profilschaft (26; 70) zwei weitere Kammern (30, 32; 74, 76) aufweist, die den sich längs erstreckenden Zuführkanal (22) und/oder den sich längs erstreckenden Abführkanal (24) bilden.

2. Endoskop nach Anspruch 1, **dadurch gekennzeichnet, daß** der Außenschaft (12) einen kreisförmigen Querschnitt aufweist, und daß eine konvexe Außenseite (46) des Profilschafts (26; 70), die an dem Außenschaft (12) anliegt, sich bis über die Hälfte des Innenumfangs des Außenschafts (12) erstreckt.

3. Endoskop nach Anspruch 1 oder 2, **dadurch gekennzeichnet, daß** einzelne der Kammern (28 - 36; 72 - 80) im Querschnitt nicht kreisförmig sind.

4. Endoskop nach einem der Ansprüche 1 bis 3, **dadurch gekennzeichnet, daß** der Profilschaft (26; 70) aus dem Außenschaft (12) herausnehmbar ist.

5. Endoskop nach einem der Ansprüche 1 bis 4, **dadurch gekennzeichnet, daß** der Optikkanal (14) und der Arbeitskanal (18) einen kreisförmigen Querschnitt aufweisen, und ein Durchmesser der den Optikkanal (14) bildenden Kammer (28; 72) und ein Durchmesser des Arbeitskanals (18) für das Arbeitselement (20) auf dem gleichen Durchmesser des Außenschafts (12) angeordnet sind.

6. Endoskop nach einem der Ansprüche 1 bis 5, **dadurch gekennzeichnet, daß** der Profilschaft (26; 70) eine solche äußere Abmessung aufweist, daß zwischen dem Arbeitskanal (18), der Innenseite des Außenschafts (12) und dem Profilschaft (26; 70) zumindest ein weiterer Abführkanal (24) und/oder Zuführkanal für ein Medium ausgebildet ist.

7. Endoskop nach einem der Ansprüche 1 bis 6, **dadurch gekennzeichnet, daß** der Profilschäft (26; 70) im Querschnitt fünf Kammern (28 - 36; 72 - 80) aufweist, wobei der Optikkanal (14) durch eine mittlere Kammer (28; 72) gebildet wird, und der Zuführkanal (22) und/oder der Abführkanal (24) sowie der zumindest eine Kanal für das Beleuchtungssystem (38) durch beidseits von dem Optikkanal (14) angeordnete Kammern (30, 32, 34, 36) gebildet werden.

8. Endoskop nach einem der Ansprüche 1 bis 7, **dadurch gekennzeichnet, daß** der Profilschaft (26; 70) einstückig ausgebildet ist.

9. Endoskop nach einem der Ansprüche 1 bis 8, **dadurch gekennzeichnet, daß** der Profilschaft aus einer der Anzahl an Kammern entsprechenden Anzahl von Einzelprofilen zusammengefügt ist.

## Claims

1. An endoscope, comprising an outer shaft (12), in which an optical channel (14) for accommodating an optical system (16) is arranged, wherein in the outer shaft (12), further, a working channel (18) for accommodating a working element (20) and at least one channel for accommodating a light supplying illumination system (38) are arranged, wherein the optical channel (14) and the at least one channel for the light supplying illumination system (38) are configured as chambers (28, 34, 36; 72, 78, 80) of a profiled shaft (26; 70) chambered in cross section, which is accommodated in the outer shaft (12), and wherein an outer contour of the profiled shaft (26; 70) is configured approximately in kidney-shaped fashion, and the working channel (18) is arranged inside the outer shaft (12) and outside the profiled shaft (26; 70) and extends from a concave outer side (42) of the profiled shaft ( 26; 70) to an opposite concave inner side (44) of the outer shaft (12), wherein, further, at least one supply channel (22) extending longitudinally for a liquid medium is present in the outer shaft (12), **characterized in that** at least one discharge channel (24) extending longitudinally for discharging a liquid medium separatedly from the supply is present in the outer shaft (12), and the profiled shaft (26; 70) comprises two further chambers (30, 32; 74, 76) which form the longitudinally extending supply channel (22) and/or the longitudinally extending discharge channel (24).

2. The endoscope of claim 1, **characterized in that** the outer shaft (12) comprises a circular cross section, and a convex outer side (46) of the profiled shaft (26; 70), which clings to the outer shaft (12), extends beyond the half of the inner circumference of the outer shaft (12).

3. The endoscope of claim 1 or 2, **characterized in that** some of the chambers (28- 36; 72 - 80) are not circular in their cross sections.

4. The endoscope of anyone of claims 1 through 3, **characterized in that** the profiled shaft ( 26; 70) is removable out of the outer shaft (12).

5. The endoscope of anyone of claims 1 through 4, **characterized in that** the optical channel (14) and the working channel (18) have a circular cross section, and a diameter of the chamber (28; 72) forming the optical channel (14) and a diameter of the working channel (18) for the working element (20) are arranged on the same diameter of the outer shaft (12).

6. The endoscope of anyone of claims 1 through 5, **characterized in that** the profiled shaft (26; 70) has such an outer dimension that between the working channel (18), the inner side of the outer shaft (12) and the profiled shaft (26; 70) at least one further discharge channel (24) and/or supply channel for a medium is formed.

7. The endoscope of anyone of claims 1 through 8, **characterized in that** the profiled shaft (26; 70) has in its cross section at least five chambers (28 - 36; 72 - 80), wherein the optical channel (14) is formed by an intermediate chamber (28; 72) and the supply channel (20) and/or the discharge channel (24) and the at least one channel for the illumination system (38) are formed by chambers (30, 32, 34, 36) arranged on both sides of the optical channel (14).

8. The endoscope of anyone of claims 1 through 7, **characterized in that** the profiled shaft (26; 70) is configured in one piece.

9. The endoscope of anyone of claims 1 through 10, **characterized in that** the profiled shaft is assembled of a number of individual profiles corresponding to the number of chambers.

## Revendications

1. Endoscope, avec une tige externe (12), dans laquelle est disposé un canal optique (14) recevant un système optique (16), la tige externe (12) comprenant en outre un canal de travail (18) pour le logement d'un élément de travail (20) et au moins un canal pour le logement d'un système d'éclairage (38) destiné à conduire la lumière, le canal optique (14) et au moins un canal pour le système d'éclairage (38) destiné à conduire la lumière étant configurés comme des chambres (28, 34, 36 ; 72, 78, 90) d'une tige profilée (26 ; 70) logée dans la section transversale, la tige profilée (26 ; 70) étant reçue dans la tige externe (12) et un contour externe de la tige profilée (26 ; 70) étant configuré en forme approximative de croissant, et le canal de travail (18) étant disposé à l'intérieur de la tige externe (12) et à l'extérieur de la tige profilée (26 ; 70) et s'étendant d'une face externe concave (42) de la tige profilée (26 ; 70) à une face interne concave opposée (44) de la tige externe (12), en outre au moins un canal de distribution (22) s'étendant longitudinalement pour un fluide liquide étant prévu dans la tige externe (12), **caractérisé en ce qu'**il est prévu dans la tige externe (12) au moins un canal d'évacuation (24) s'étendant longitudinalement pour l'évacuation séparée par rapport à la distribution d'un fluide liquide, et **en ce que** la tige profilée (26 ; 70) comprend deux autres chambres (30, 32 ; 74, 76) qui forment le canal de distribution (22) s'étendant longitudinalement et/ou le canal d'évacuation (24) s'étendant longitudinalement.

2. Endoscope selon la revendication 1, **caractérisé en ce que** la tige externe (12) comporte une section transversale circulaire, et **en ce qu'**une face externe (46) convexe de la tige profilée (26 ; 70), qui est adjacente à la tige externe (12), s'étend jusqu'au-delà de la moitié du périmètre interne de la tige externe (12).

3. Endoscope selon la revendication 1 ou 2, **caractérisé en ce que** les différentes chambres (28-36 ; 72-80) présentent une section transversale non circulaire.

4. Endoscope selon l'une des revendications 1 à 3, **caractérisé en ce que** la tige profilée (26 ; 70) peut être extraite de la tige externe (12).

5. Endoscope selon l'une des revendications 1 à 4, **caractérisé en ce que** le canal optique (14) et le canal de travail (18) comportent une section transversale circulaire, et un diamètre de la chambre (28 ; 72) formant le canal optique (14) et un diamètre du canal de travail (18) pour l'élément de travail (20) sont disposés sur le même diamètre de la tige externe (12).

6. Endoscope selon l'une des revendications 1 à 5, **caractérisé en ce que** la tige profilée (26 ; 70) comporte une dimension externe telle qu'au moins un autre canal d'évacuation (24) et/ou un canal de distribution pour un fluide sont formés entre le canal de travail (18), la face interne de la tige externe (12) et la tige profilée (26 ; 70).

7. Endoscope selon l'une des revendications 1 à 6, **caractérisé en ce que** la tige profilée (26 ; 70) comprend cinq chambres (28-36 ; 72-80) dans sa section transversale, le canal optique (14) étant formé par une chambre intermédiaire (28 ; 72) et le canal de distribution (22) et/ou le canal d'évacuation (24), ainsi qu'au moins un canal pour le système d'éclairage (38) étant formés par des chambres (30, 32, 34, 36) disposées des deux côtés du canal optique (14).

8. Endoscope selon l'une des revendications 1 à 7, **caractérisé en ce que** la tige profilée (26 ; 70) est configurée en une seule pièce.

9. Endoscope selon l'une des revendications 1 à 8, **caractérisé en ce que** la tige profilée est composée d'un nombre de profilés individuels correspondant au nombre de chambres.
